Europäisches Patentamt

European Patent Office

Office européen des brevets

(19)

(11) Numéro de publication : **0 338 950 B1**

(12) **FASCICULE DE BREVET EUROPEEN**

(45) Date de publication du fascicule du brevet :
**18.08.93 Bulletin 93/33**

(51) Int. Cl.$^5$ : **C02F 3/34, C12R 1/72**

(21) Numéro de dépôt : **89420141.7**

(22) Date de dépôt : **19.04.89**

(54) **Procédé d'épuration et de valorisation, par voie microbiologique, d'un effluent organique comprenant un sucre fermentescible.**

(30) Priorité : **20.04.88 FR 8805711**

(43) Date de publication de la demande :
**25.10.89 Bulletin 89/43**

(45) Mention de la délivrance du brevet :
**18.08.93 Bulletin 93/33**

(84) Etats contractants désignés :
**AT BE CH DE ES FR GB GR IT LI LU NL SE**

(56) Documents cités :
**AT-C- 376 956**
**FR-A- 2 280 588**
**GB-A- 2 023 118**
**Biotechnology, H.-J. Rehm / G. Reed, Verlag Chemie, Vol. 3 pages 35-36, Deutsche Sammlung von Mikroorganismen, Cataloge of Strains, 3rd Ed., 1983.**

(73) Titulaire : **KEFIRANA**
**Montée de la Croix d'Evieu Saint Clair de la Tour**
**F-38110 La Tour du Pin (FR)**

(72) Inventeur : **Grandjacquet, Louis**
**Montée de la Croix d'Evieu Saint Clair de la Tour**
**F-38110 La Tour Du Pin (FR)**

(74) Mandataire : **Guerre, Dominique et al**
**Cabinet Germain et Maureau Le Britannia-Tour C 20 Boulevard Eugéne Deruelle BP 3011**
**F-69392 Lyon Cédex 03 (FR)**

## Description

La présente invention concerne un procédé d'épuration et de valorisation par voie microbiologique d'un effluent organique comprenant un sucre fermentescible, en particulier d'effluents de ce type résultant d'activités agroalimentaires, agricoles, ou industrielles.

Par épuration, on entend le fait de diminuer dans une proportion d'au moins 50 % la demande chimique en oxygène (DCO) de l'effluent traité.

Par valorisation, on entend le fait que l'épuration génère des produits, notamment biomasse, différents de ceux constituant l'effluent traité, ayant en soi une utilité, notamment alimentaire.

S'agissant de l'épuration microbiologique d'effluents, l'art antérieur a déjà proposé la mise en oeuvre en conditions aérobies de co-cultures d'au moins deux levures, s'installant dans un milieu de culture contenant l'effluent à traiter ou épurer.

Ainsi, selon le brevet GB-A-2023118, avec une co-culture de levures des genres Trichosporon et Candida respectivement, on propose d'épurer des effluents résultant de la fabrication d'huile de palme, c'est-à-dire de substrats constitués principalement de composés lipidiques et de saccharides non identifiés.

Parmi les espèces de levures citées pour le genre Trichosporon seule l'espèce Cutaneum assimile le lactose, mais dans une mesure limitée. Quant aux espèces citées pour le genre Candida, aucune n'assimile du lactose. Ces co-cultures apparaissent donc spécifiques au traitement d'effluents lipidiques, et donc incapables pour l'essentiel de métaboliser du lactose par exemple, pour la production d'une biomasse correspondante.

Selon le brevet AT-C-376956, pour la dépollution d'eaux résiduaires, en milieu ouvert, on a proposé de mettre en oeuvre une co-culture de levures appartenant au genre, Candida. La seule indication du genre de la levure apparaît insuffisant pour déterminer sa capacité à assimiler un substrat donné, par exemple un sucre fermentescible. Il s'agit par ailleurs d'un procédé de dépollution traditionnel avec production d'un floc du type boues activées, sans spécificité particulière.

S'agissant d'effluents contenant du lactose, et plus précisément d'effluents de fromageries du type lactoserum brut, selon le brevet FR-A-2492403, on a décrit la mise en oeuvre d'une co-culture justaposant au moins quatre espèces différentes de levures se développant indépendamment les unes des autres, la première, Kluyveromyces fragilis assimilant le lactose, la seconde Kluyveromyces lactis assimilant l'acide lactique, la troisième, Candida utilis ou Saccharomyces cerevisae, assimilant le glucose, et la quatrième, Torulopsis bovina, assimilant l'éthanol.

L'effluent à épurer, doit subir différents traitements avant de participer au milieu de culture :
- il doit tout d'abord être dilué, car la co-culture mise en oeuvre ne supporte pas directement, sans inconvénients importants (diminution du rendement), la forte charge organique de l'effluent brut ; pour du lactoserum brut, ces charges sont de l'ordre de 60-70 grammes de DCO par litre, résultant principalement de 40 à 50 grammes de lactose par litre selon le procédé de transformation du lait.
- il doit être ensuite déprotéiné, par précipitation à chaud au point isoélectrique de l'effluent ou par ultrafiltration
- il doit être enfin pasteurisé, voire stérilisé pour éviter tout développement dans le milieu de culture de la charge microbienne originelle du lait traité, ou des ferments employés lors de la transformation du lait.

En définitive, un tel procédé nécessite des équipements et appareillages nombreux, complexes pour certains d'entre eux, et en tous cas gros consommateurs d'énergie. Sa rentabilité passe donc par le traitement de volumes très importants.

La présente invention a pour objet un procédé d'épuration et valorisation par voie microbiologique d'un effluent comprenant un sucre fermentescible, permettant de traiter directement l'effluent, c'est-à-dire sans opération particulière préalable à l'introduction dudit effluent dans le milieu de culture.

Selon la présente invention, on a découvert qu'on pouvait traiter directement, c'est-à-dire sans traitement préalable, les effluents considérés par la présente invention, en utilisant une co-culture constituant un système microbiologique métabolisant l'effluent, et associant de manière compatible :
- une souche d'une première levure appartenant à l'espèce Candida Kefyr, métabolisant le sucre fermentescible en générant un sous-produit, notamment de l'éthanol
- et une autre souche d'une deuxième levure appartenant à l'espèce Candida Valida, métabolisant sans diauxie le sous-produit.

Préférentiellement, les souches des première et seconde levures du système microbiologique selon la présente invention sont respectivement les souches déposées auprès de la Collection Nationale des Micro-organismes de l'Institut Pasteur, sous les références I-747 et I-748 respectivement.

La première souche de levure, d'une part assure une métabolisation relativement rapide du sucre fermentescible choisi à titre de substrat dans l'effluent, et d'autre part présente une activité fermentaire résiduelle vis-à-vis du même sucre, générant au moins un sous-produit pouvant inhiber sa croissance à des concentrations relativement importantes.

Par métabolisation relativement rapide, on entend le fais que le système microbiologique selon l'invention peut être mis en oeuvre directement, c'est-à-dire sans dilution préalable, avec un taux de croissance global en culture continue d'au moins 0,20 h$^{-1}$.

La seconde couche de levure assure sans diauxie une métabolisation du sous-produit de fermentation résiduelle de la première levure.

A titre d'exemple, le substrat retenu étant le lactose, la première levure assimile relativement vite le lactose, et génère au moins de l'éthanol ; de l'acide lactique résulte aussi de l'activité métabolique de la première souche et/ou est apporté par l'effluent. Et la seconde levure assimile sans diauxie l'éthanol et l'acide lactique éventuellement apporté par l'effluent traité.

Les deux espèces de levures du système microbiologique selon l'invention interagissent l'une vis à vis de l'autre de manière synergique, de la manière suivante :

- la première espèce, en raison de son assimilation rapide du sucre fermentescible, génère des sous-produits de fermentation résiduelle, qui s'ils demeuraient dans le milieu de culture conduiraient à la stagnation voire la régression de la biomasse
- la seconde espèce, en raison de son assimilation simultanée des sous-produits de fermentation résiduelle détoxifie en permanence le milieu de culture, accroît et stimule de ce fait la métabolisation exercée par la première espèce.

Au total, le système microbiologique selon l'invention permet d'assimiler directement des charges organiques relativement fortes, par exemple de l'ordre de 60 à 70 Kg de DCO/m$^3$, tout en métabolisant les principaux substrats carbonés polluants présents dans cette charge, y compris ceux générés au sein du milieu de culture. On obtient selon l'invention des rendements de conversion et donc d'épuration relativement élevés, par exemple de l'ordre de 50 % et 90 % respectivement, tout en produisant une biomasse de bonne qualité, et donc valorisable.

Par rapport à tout effluent comprenant un sucre fermentescible à transformer et valoriser, l'homme de métier est à même de définir un système microbiologique selon l'invention, par l'exécution de tests simples, tels qu'identifiés dans les tableaux I et II ci-après, ce qui lui permet de sélectionner deux espèces de levures actives, présentant des propriétés métaboliques analogues à celles des souches déposées auprès de l'Institut Pasteur respectivement sous les références 1-747 et 1-748, et précisées pour l'essentiel dans les tableaux I et II. Les levures ainsi choisies devront en outre être compatibles l'une par rapport à l'autre, en termes de non compétition entre elles.

Avec un système microbiologique selon l'invention, on est à même de métaboliser des effluents contenant des sucres simples fermentescibles comme le lactose, le glucose, le galactose, mais aussi des sucres complexes fermentescibles comme le saccacharose, le xylose, l'arabinose, et le fructose. Le milieu de culture peut en outre contenir à titre de substrat, apporté par l'effluent ou généré par la culture, de l'éthanol, de l'acide lactique, de l'acide succinique, de l'acide citrique, et du glycerol.

Le système microbiologique apporte en outre les avantages déterminants suivants, et ce de manière inattendue.

Le rendement de conversion s'avère relativement insensible à la concentration du sucre fermentescible de l'effluent, et donc à sa dilution. Ceci est lié à la valeur élevée de la constante d'inhibition (Ki) de la première souche retenue vis à vis du sucre fermentescible, et donc à l'absence d'effet CRABTREE (inhibition du métabolisme aérobie par une forte concentration en sucre).

Il résulte des caractéristiques métalobiques et physiologiques des deux souches du système selon l'invention, explicitées précédemment, que celles-ci peuvent s'installer de manière sélective dans le milieu de culture comprenant l'effluent, vis à vis des autres microorganismes présents dans ce dernier. Ceci conduit à une stabilité et une pérennité en culture du système microbiologique selon l'invention.

L'interaction des deux souches, caractérisant la synergie du système microbiologique selon l'invention, évite tout phénomène de compétition entre les deux levures, et donc tout risque de supplantation de l'une par l'autre. Ceci contribue encore à la stabilité en culture selon l'invention, et permet plus de souplesse quant à la qualité des substrats traités.

Un système microbiologique selon l'invention s'avère très résistant à des pH très acides. Ceci permet d'opérer dans des conditions homeostatiques, exerçant une action bactériostatique voire bactéricide vis à vis des microflores d'origine endogène ou exogène présentes dans l'effluent à traiter.

Un système microbiologique selon l'invention peut être mis en oeuvre en milieu homogène, par exemple dans un fermenteur, c'est-à-dire dans des conditions de transfert optimal de l'oxygène et des substrats vers les levures.

Ainsi, un procédé selon l'invention peut comporter :

- une étape de fonctionnement en discontinu séquentielle, consistant à installer dans le milieu de culture, par exemple dans un fermenteur, successivement la première souche, puis la deuxième souche
- puis une étape de culture proprement dite, notamment continue, mise en oeuvre par exemple dans le fermenteur.

Ces deux étapes sont suivies d'une étape de séparation de la biomasse produite, par ultra-filtration, microfiltration ou centrifugation, cette biomasse étant ensuite traitée et conditionnée en vue de son utilisation ultérieure.

S'agissant du système <u>Candida Kefyr/Candida valida</u>, on peut encore préciser les conditions opératoires suivantes caractérisant l'invention.

Selon un mode préféré, les conditions de culture discontinue et continue sont choisies de façon à ce que la température soit comprise entre 30 et 38°C, le pH entre 2 et 4,5 unités et le débit entre 1 et 2 VVM (Volume d'air par volume de milieu de culture traité et par minute).

Au cours de l'étape de culture en continu, l'alimentation en effluent se fait à un débit F constant pour un volume utile V d'effluent contenu dans le fermenteur. Un système de régulation commande la sortie de milieu de culture de telle sorte que le taux de dilution F/V soit stabilisé a une valeur inférieure à O,5O h$^{-1}$.

Cette disposition permet de traiter rapidement de grandes quantités d'effluents.

De préférence, le taux de dilution F/V est modulable en fonction de la quantité et de la qualité des substrats à traiter.

Afin d'optimiser le développement des souches, le substrat à traiter est complémenté avec une source d'azote minéral, par exemple sous forme de $(NH_4)_2 SO_4$, des oligoéléments comprenant, entre autres, du Co, Fe, Mn, Zn et des facteurs de croissance, par exemple sous forme d'extraits de levure ou de "Corn Steep Liquor".

D'autres avantages et caractéristiques de l'invention apparaîtront à l'aide de la description qui suit, d'exemples non limitatifs de mise en oeuvre du procédé d'épuration et de valorisation d'effluents organiques tels que les lactosérums de toutes natures, notamment le lactosérum brut.

En premier lieu, il est nécessaire de décrire précisément un système microbiologique selon l'invention.

La première souche, déposée le 14 Avril 1988 à la Collection Nationale de Cultures de Microorganismes de l'Institut Pasteur sous la référence 1-747, est identifiée à <u>Candida Kefyr</u>. Ces caractéristiques morphologiques et biochimiques sont illustrées par le Tableau I qui suit.

## TABLEAU I

| ASSIMILATION | | FERMENTATION | |
|---|---|---|---|
| Substrat | Résultats | | |
| | | | |
| Glucose | + | + | |
| Galactose | + | + | |
| Saccharose | + | + | |
| Maltose | - | - | |
| Cellobiose | + d | | |
| Lactose | (+) | + | |
| Raffinose | + | W | |
| Amidon soluble | - | | |
| Xylose | + d | TESTS COMPLEMENTS | |
| L-Arabinose | W | | |
| Ribose | - | Croissance à 37°C : - | |
| Rhamnose | - | Croissance à 43°C : - | |
| Erythritol | - | | |
| Mannitol | | Choline : - | |
| Acide succinique | W | L.Lysine : - | |
| Acide citrique | - | Creatinine : - | |
| Inositol | - | D.glucose-amine : - | |
| Ethanol | + | Croissance 2% glucose : - | |
| Glycerol | + d | Croissance 60% glucose : - | |
| Nitrate | - | | |
| Nitrite | - | Hydrolyse de l'urée : - | |
| Sans vitamines | - | | |
| Sorbose | - | | |
| Melibiose | - | | |
| Melezitose | - | | |
| Inuline | + | Croiss. sans thiamine : - | |
| D-Arabinose | - | Croiss. sans biotine : + | |
| Glucosamine | - | Croiss. sans pyridoxine : - | |
| Sorbitol | + | Croiss. sans niacine : - | |
| ⍺-methyl glucoside | - | Croiss. sans inositol : - | |
| Salicine | + | Croiss. sans PAB : + | |
| Acide lactique | + | Croiss. sans ac. folique : - | |
| Arbutine | - | Croiss. sans pantothenate : - | |
| | | Croiss. sans riboflavine : - | |
| AUTRES OBSERVATIONS | | Levure de forme ovoïde 3 à 5 µm/4 à 5 µm | |
| | | bourgeonnement multi-lateral | |
| La souche est identifiée à | | SPORULATION | |
| | | Gorodkowa : - | |
| Candida kefyr | | Gasell : - | |
| | | LAMES GELOSEES | |
| | | pseudomycelium : - | |
| | | vrai mycelium : - | |

La seconde souche, déposée le 14 Avril 1988 à la Collection Nationale de Cultures de microorganismes de l'Institut Pasteur sous la référence 1-748, est identifiée à <u>Candida valida</u>. Ces caractéristiques morphologiques et biochimiques sont données par le Tableau II qui suit.

## TABLEAU   II

| ASSIMILATION | | FERMENTATION |
|---|---|---|
| Substrat | Résultats | |
| | | |
| Glucose | + | + W |
| Galactose | - | - |
| Saccharose | - | - |
| Maltose | - | - |
| Cellobiose | - | - |
| Lactose | - | - |
| Raffinose | - | - |
| Amidon soluble | - | - |
| Xylose | W | **TESTS COMPLEMENTS** |
| L-Arabinose | - | |
| Ribose | - | Croissance à 37°C :  - |
| Rhamnose | - | Croissance à 45°C : - |
| Erythritol | - | |
| Mannitol | - | Choline :  - |
| Acide succinique | - | L-Lysine :  - |
| Acide citrique | + | Creatinine :  - |
| Inositol | - | D.glucose-amine : - |
| Ethanol | - | Croissance 20 %glucose  : - |
| Glycerol | - | Croissance 60 %glucose  : - |
| Nitrate | - | |
| Nitrite | - | Hydrolyse de l'Urée : - |
| Sans vitamines | - | |
| Sorbose | - | |
| Melibiose | - | |
| Melezitose | - | |
| Inuline | - | Croiss. sans thiamine : - |
| D-Arabinose | - | Croiss. sans biotine :  - |
| Glucosamine | - | Croiss. sans pyridoxine : - |
| Sorbitol | - | Croiss. sans niacine :  - |
| α-methyl glucoside | - | Croiss. sans inositol :  - |
| Salicine | - | Croiss. sans PAB :  - |
| Acide lactique | - | Croiss. sans ac. folique :  - |
| Arbutine | - | Croiss. sans pantothenate : - |
| : | | Croiss. sans riboflavine :  + |
| **AUTRES OBSERVATIONS** | | Levure de forme allongée 10 à 15 µm/4 à 6 µm Bourgeonnement multi-lateral |
| La souche est identifiée à | | SPORULATION Gorodkowa : - |
| Candida valida | | Fowel : - |
| . | | LAMES GELOSEES Pseudomycelium :  - Vrai mycelium :  - |

Dans l'exemple décrit, ces souches sont conservées sur milieu Yeast Malt, à 4°C. Elles sont repiquées tous les six mois sur ce même milieu, en cinq exemplaires, lesquels serviront de stoks pour la réalisation des précultures ou levains pour la mise en oeuvre du procédé.

Ces précultures sont réalisées séparément pour chaque souche. La souche Candida Kefyr est incubée 24 heures à 35°C sur un substrat laitier, c'est-à-dire du lactosérum brut, complémenté par trois pour mille en volume de sulfate d'ammonium $(NH_4)_2 SO_4$, par O,2 pour mille d'extrait de levure et par 1ml/l d'une solution d'oligoéléments selon BAYER $(CuSO_4 , MnSO_4 ; Na_2 MoO_4 ; FeCl_3)$, le pH de ce milieu est amené à 4,5 par ajout d'acide chlorydrique 1N. Il en va de même pour la souche Candida valida, à la différence près, que l'on ajoute de l'éthanol selon une concentration de 10 % en volume dans le lactosérum brut complémenté.

Après centrifugation, les précultures de chaque souche sont reprises dans des volumes minimums de lactosérum brut complémenté, exempt d'éthanol, afin de constituer un premier inoculum de Candida Kefyr et un second inoculum de Candida valida.

Conformément au procédé selon l'invention, vient ensuite, l'étape de fonctionnement discontinue séquentielle. Elle se déroule dans un fermenteur "Chemostat" du type BIOLAFITTE, connu en soi, suivant deux phases ou séquences déterminées.

Dans un premier temps, un volume utile V de lactosérum brut complémenté placé dans le fermenteur est ensemencé par l'inoculum de Candida Kefyr. La température de culture est comprise entre 30°C et 38°C, le pH entre 2 et 4,5 régulé par de l'hydroxyde d'ammonium $(NH_4OH)$ 5N. Le débit d'air nécessaire est de l'ordre de 1 à 2 VVM (Volume d'air par volume de milieu traité et par minute) et une agitation comprise entre 600 et 800 tours par minute est entretenue à l'intérieur du fermenteur.

Dans un deuxième temps, dès l'apparition d'éthanol dans le milieu de culture, on procède à l'ensemencement de l'inoculum de Candida valida dans le fermenteur.

La deuxième phase de cette culture discontinue est une culture du système microbiologique des deux souches considérées. Lorsque, dans cette culture, les deux microorganismes considérés ont atteint leur phase exponentielle de croissance depuis plus de deux heures, l'étape de culture continue peut débuter.

Le fermenteur est alors alimenté constamment en substrat laitier brut complémenté. Ce dernier est conservé dans une enceinte de stockage réfrigérée à 4°C, d'où il est soutiré en continu à un débit F par l'intermédiaire d'une pompe d'alimentation. Le taux de dilution correspondant à F/V, et inférieur à O,5O h⁻¹, est maintenu constant, par exemple égal à O,2O h⁻¹, par un système de régulation comprenant un dispositif de pesée commandant le fonctionnement d'une pompe d'évacuation du milieu de culture.

Les conditions de culture, température, agitation, aération, pH sont identiques à celles décrites précédemment pour l'étape de culture discontinue.

Au terme du procédé, la biomasse protéique produite est récupérée avec les grosses molécules présentes à l'origine dans l'effluent, telles que protéines, enzymes, polypeptides, par ultrafiltration, microfiltration, ou toute autre méthode appropriée. Elle peut être également récupérée seule par centrifugation. Elle est ensuite concentrée sous forme d'une pâte, puis soumise à une thermolyse, c'est-à-dire à un choc thermique destiné à inactiver les microorganismes et à augmenter la digestibilité des levures. Cette pâte est enfin stockée, broyée puis conditionnée.

Les exemples suivants précisent l'invention, sans toutefois la limiter.

Le premier exemple concerne l'application du procédé à un lactosérum brut doux issu d'une fabrication d'emmental. Sa composition initiale est la suivante :

Lactose     4,5 ± 3g.l⁻¹
Citrate      1,7 ± O,5g.l⁻¹
Lactate     O,3 ± O,1g.l⁻¹

Le traitement suivant le procédé selon l'invention de ce substrat s'effectue dans les conditions suivantes :

- Complémentation nutritionnelle du lactosérum : $(NH_4)^2SO_4)$ (4,5g.l⁻¹), extrait de levure (1g.l⁻¹), solution d'oligoéléments selon BAYER (1ml l⁻¹) ; $CuSO_4 - 5H_2O$ (O,3g.l⁻¹) ; $MnSO_4 - H_2O$(O,8g.l⁻¹) ; $Na_2MoO_4 - 2 H_2O$ (O,4g.l⁻¹) ; $ZnSO_4 - 7H_2O$ (3g.l⁻¹) ;$FeCl_3 - 6 H_2O$ (4g.l⁻¹).
- Taux de dilution = O,2O h⁻¹
- pH = 2 à 4,5, régulation par $NH_4OH$
- Température = 30 à 38°C
- Aération = 1,5 VVM.

Les performances obtenues sont les suivantes :

Les contrôles à l'équilibre ont montré une répartition très stable entre les deux souches Candida Kefyr et Candida valida représentant respectivement de 60 % à 40 % et de 40 % à 60 % de la population totale.

a) Epuration :

Abattement de la demande chimique en oxygène (DCO) supérieur à 97 %:

Lactose résiduel      inférieur à O,1g.l$^{-1}$
Citrate résiduel      inférieur à O,1g.l$^{-1}$
Lactate résiduel      inférieur à O,1g.l$^{-1}$

b) Valorisation :

Biomasse produite à l'équilibre 21 ± 3 Kg.m$^{-3}$, soit un rendement en biomasse de l'ordre de 53 %.

Le second exemple concerne l'application du procédé à un perméat laitier brut dont la composition initiale est la suivante :

Lactose    41 ± 3g.l$^{-1}$
Citrate    1,5 ± O,5g.l$^{-1}$
Lactate    O,5 ± O,1g.l$^{-1}$

Le traitement par le procédé selon l'invention de ce substrat s'effectue dans les conditions suivantes :

- Complémentation nutritionnelle du perméat : $((NH_4)_2SO_4)$ (4,5g.l$^{-1}$), extrait de levure (1g.l$^{-1}$), solution d'oligoéléments selon BAYER (1ml.l$^{-1}$) ; $CuSO_4$ - $5H_2O$ (O,3g.l$^{-1}$) ; $MnSO_4$ $H_2O$(O,8 g.l$^{-1}$) ; $Na_2MoO_4$ - 2 $H_2O$ (O,4g.l$^{-1}$) ; $ZnSO_47H_2O$ (3g.l$^{-1}$) ; $FeCl_3$ - 6 $H_2O$ (4g.l$^{-1}$).
- Taux de dilution = O,2O h $^{-1}$ à O,3O h-1
- pH 2,O à 4,5 régulation par $NH_4OH$
- Température = 30 à 38°C

Les performances obtenues sont les suivantes :

Les contrôles à l'équilibre ont montré une répartition très stable entre les deux souches <u>Candida Kefyr</u> et <u>Candida valida</u>, représentant respectivement de 60 à 4O % et de 40 à 60 % de la population totale.

a) Epuration :

Abattement de la demande chimique en oxygène (DCO) supérieur à 97 %.

Lactose résiduel      inférieur à O,1g.l$^{-1}$
Citrate résiduel      inférieur à O,1g.l$^{-1}$
Lactate résiduel      inférieur à O,1g.l$^{-1}$

b) Valorisation :

Biomasse produite à l'équilibre : 2O ± 2,5 Kg.m$^{-3}$

Soit un rendement en biomasse d'environ 50 %.

L'invention peut également trouver application dans l'épuration d'effluents autres que ceux résultant de l'industrie laitière, mais toujours contenant un sucre fermentescible, comme les mélasses, les saumures de confiserie ou conserverie, les drêches de brasserie, les jus de presse de fruits et d'agrumes.

Le troisième exemple concerne l'application du procédé à des jus de drêches de brasserie dont la composition initiale est la suivante :

Glucose :      5,2 ± 1,1g.l$^{-1}$
Xylose :      O,8 ± O,2g.l$^{-1}$
Arabinose :      O,8 ± O,2g.l$^{-1}$

Le traitement de cet effluent s'effectue dans les conditions suivantes :

- Complémentation nutritionnelle des jus de drêches :
  $((NH_4)_2 SO_4)$ (O,8 g.l$^4$) ; extrait de levure (O,5g/l), solution d'oligoéléments (1 ml/l)
  pH de culture : 2 à 4,5 unités, régulation par $NH_4OH$
- Température : 30 à 38°C
- Aération : 1,5 VVM

Les performances obtenues sont les suivantes :

Les contrôles à l'équilibre ont montré une répartition stable entre les deux souches, <u>Candida Kefyr</u> et <u>Candida valida</u>, représentant respectivement de 80 à 60 % et de 20 à 40 % de la population totale.

a) Epuration :

Abattement de la demande chimique en oxygène (DCO) supérieur à 90 %, avec un rendement en biomasse supérieur à 46 %.

Glucose résiduel      inférieur à O,2g.l$^{-1}$
Xylose résiduel      inférieur à O,2g.l$^{-1}$

Arabinose résiduel    inférieur à O,2g.l$^{-1}$

b) Valorisation :

Biomasse produite à l'équilibre 3,2 ± O,5 Kg.m$^3$

Soit un rendement en biomasse de 47 %.

Le quatrième exemple concerne l'application du procédé à des vinasses de mélasse de betteraves, dont la composition initiale est la suivante :

Sucres totaux        26,O ± 3,2g.l$^{-1}$
Sucres réducteurs    18,3 ± 2,1g.l$^{-1}$
Acide acétique       5,6 ± 1,1g.l$^{-1}$
Acide lactique       7,0 ± 1,1g.l$^{-1}$
Glycérol             7,O ± O,9g.l$^{-1}$

Le traitement de cet effluent s'effectue dans les conditions suivantes :

- complémentation nutritionnelle des mélasses :

    $((NH_4)_2SO_4)$ (4g.l$^{-1}$) ; extrait de levure (1g.l$^{-1}$) ; solution d'oligoéléments (1ml.l$^{-1}$).

- pH de culture : 2 à 4,5 unités
- Température : 30 à 38°C
- Aération : 1,5 VVM

Les performances obtenues sont les suivantes :

Les rapports des populations à la population totale atteignaient 50 à 20 % pour <u>Candida Kefyr</u> et 50 ± 20 % pour <u>Candida valida</u>.

a) Epuration :

L'épuration est supérieure à 85 % d'abattement de la DCO.

Sucres résiduels    inférieurs à 2,5g.l$^{-1}$
Acide acétique      inférieur à O,7 g.l$^{-1}$
Acide lactique      inférieur à O,2g.l$^{61}$
Glycérol            inférieur à O,2g.l$^{-1}$

b) Valorisation :

Biomasse produite : 19 ± 2 Kg.m$^{-3}$

Soit un rendement en biomasse d'environ 50 %.

Un cinquième exemple concerne les saumures de confiserie, dont la composition initiale est la suivante :

Sucres totaux        20 ± 2,2g.l$^{-1}$
Sucres réducteurs    16,5 ± 1,5g.l$^{-1}$
soit Glucose         8,5 ± 1,1g.l$^{-1}$
Fructose             6,1 ± 0,7g.l$^{-1}$

Le traitement s'effectue dans les conditions suivantes :

- Complémentation nutritionnelle des saumures :

    $((NH_4)_2 SO_4)$ (2g.l$^{-1}$) ; extrait de levure (1g.l$^{-1}$) ; solution d'oligoéléments (1 ml.l$^{-1}$).

- pH de culture : 2 à 4,5 unités
- Température : 30 à 38°C
- Aération : 1,5 VVM.

Les performances obtenues sont les suivantes :

Les rapports des populations à la population totale atteignaient 50 ± 20 % pour <u>Candida Kefyr</u> et 50 ± 2O % pour <u>Candida valida</u>.

a) Epuration :

L'épuration est supérieure à 90 % d'abattement de la DCO, la tenue en sucres résiduels étant inférieure à 5g/l$^{-3}$

b) Valorisation :

Biomasse produite 10 ± 2g.l$^{-1}$

**10**

Soit un rendement en biomasse d'environ 50 %.

## Revendications

1. Procédé d'épuration et valorisation d'un effluent organique comprenant un sucre fermentescible, consistant à mettre en oeuvre en conditions aérobies, une co-culture d'au moins deux levures s'installant de manière dominante dans un milieu de culture, une première levure métabolisant au moins le sucre fermentescible, en générant au moins un sous-produit inhibant sa croissance, et une seconde levure métabolisant au moins ledit sous-produit, **caractérisé en ce que** la co-culture est un système microbiologique métabolisant l'effluent sans traitement préalable de ce dernier, et associant de manière compatible une souche de la première levure appartenant à l'espèce Candida kefyr, métabolisant le sucre fermentescible en générant un sous-produit, notamment de l'éthanol, et une souche de la deuxième levure appartenant à l'espèce Candida valida, métabolisant sans diauxie le sous-produit.

2. Procédé selon la revendication 1, caractérisé en ce que le surcre fermentescible appartient à la liste suivante, à savoir du lactose, du glucose, du galactose, du saccharose, du xylose, de l'arabinose, du fructose.

3. Procédé selon la revendication 1, caractérisé en ce que l'effluent traité contient, en outre, au moins un composé appartenant à la liste suivante, à savoir éthanol, acide succinique, acide citrique, acide lactique, glycérol.

4. Procédé selon la revendication 1, caractérisé en ce que la culture est mise en oeuvre dans un fermenteur, la biomasse produite étant séparée par ultra-filtration, microfiltration ou centrifugation.

5. Procédé selon la revendication 1, caractérisé en ce qu'il comporte une étape de fonctionnement en discontinu séquentiel, consistant à installer dans le milieu de culture la première souche, puis la deuxième souche, suivie d'une étape de culture notamment continue.

6. Procédé selon la revendication 1, caractérisé en ce que le milieu de culture est mis en oeuvre avec au moins une des conditions spécifiques suivantes, à savoir :
   - la température est comprise entre 30°C et 38°C
   - le pH est compris entre 2 et 4,5.

7. Procédé selon la revendication 1, l'effluent organique traité étant un sous-produit de l'industrie laitière, tel qu'un lactoserum brut ou un perméat laitier brut, caractérisé en ce que ledit effluent, sans traitement préalable, est mis au contact de la co-culture.

8. Levure de l'espèce Candida kefyr, identique à la souche déposée à la Collection Nationale de Micro-Organismes de l'Institut Pasteur, sous la référence I-747.

9. Levure de l'espèce Candida valida, identique à la souche déposée à la Collection Nationale de Micro-Organismes de l'Institut Pasteur sous la référence I-748.

## Patentansprüche

1. Verfahren zur Reinigung und Aufwertung eines organischen, einen vergärbaren Zucker enthaltenden Abwassers, bestehend aus Einsetzen einer Co-Kultur von zumindest zwei Hefen unter anaeroben Bedingungen, die sich in einem Nährmedium dominant etablieren, und zwar einer ersten Hefe, die zumindest den vergärbaren Zucker metabolisiert, wobei zumindest ein Nebenprodukt erzeugt wird, das deren Wachstum hemmt, und einer zweiten Hefe, die zumindest das Nebenprodukt metabolisiert, dadurch gekennzeichnet, daß die Co-Kultur ein mikrobiologisches System ist, das das Abwasser ohne Vorbehandlung desselben metabolisiert, und das auf kompatible Art und Weise einen Stamm der ersten Hefe, der der Spezies Candida kefyr zugehörig ist, und der den vergärbaren Zucker unter Bildung eines Nebenproduktes, insbesondere Ethanol, metabolisiert, und einen Stamm der zweiten Hefe assoziiert, der der Spezies Candida valida zugehörig ist, und der das Nebenprodukt ohne Diauxie metabolisiert.

2. Verfahren nach Anspruch 1, dadurch gekennzeichnet, daß der vergärbare Zucker der folgenden Aufzählung zugehörig ist, nämlich Lactose, Glucose, Galactose, Saccharose, Xylose, Arabinose, Fructose.

3. Verfahren nach Anspruch 1, dadurch gekennzeichnet, daß das behandelte Abwasser darüber hinaus zumindest eine Verbindung enthält, die in der folgenden Auflistung erscheint, nämlich Ethanol, Bernsteinsäure, Zitronensäure, Milchsäure, Glycerol.

4. Verfahren nach Anspruch 1, dadurch gekennzeichnet, daß die Kultur in einen Fermenter eingesetzt wird, und daß die erzeugte Biomasse durch Ultrafiltration, Mikrofiltration oder Zentrifugieren getrennt wird.

5. Verfahren nach Anspruch 1, dadurch gekennzeichnet, daß es einen aufeinanderfolgend diskontinuierlichen Betriebsabschnitt aufweist, bestehend aus Einsetzen der ersten Hefe in das Nährmedium; anschließend der zweiten Hefe, gefolgt von einem kontinuierlichen Kultivierungsabschnitt.

6. Verfahren nach Anspruch 1, dadurch gekennzeichnet, daß das Nährmedium unter zumindest einer der folgenden speziellen Bedingungen eingesetzt wird, nämlich:
   - die Temperatur wird zwischen 30 und 38°C gehalten
   - der pH-Wert liegt zwischen 2 und 4,5.

7. Verfahren nach Anspruch 1, bei dem das behandelte organische Abwasser ein Abfallprodukt der Milchindustrie, wie beispielsweise eine Rohmolke oder ein Rohmilchpermeat, ist, dadurch gekennzeichnet, daß das Abwasser, ohne Vorbehandlung, mit der Co-Kultur in Berührung gebracht wird.

8. Hefe der Spezies Candida kefyr, identisch mit dem Stamm, wie er bei der *COLLECTION NATIONALE DE CULTURES DE MICRO-ORGANISMES (CNCM) Institut Pasteur* unter der Nummer I-747 hinterlegt ist.

9. Hefe der Spezies Candida valida, identisch mit dem Stamm, wie er bei der *COLLECTION NATIONALE DE CULTURES DE MICRO-ORGANISMES (CNCM) Institut Pasteur* unter der Nummer I-748 hinterlegt ist.


## Claims

1. Method for purification and exploitation of an organic effluent comprising a fermentable sugar, consisting in using, under aerobic conditions, a co-culture of at least two yeasts set up in a dominant manner in a culture medium, a first yeast metabolising at least the fermentable sugar, while generating at least one by-product which inhibits its growth, and a second yeast metabolising at least the said by-product, characterised in that the co-culture is a microbiological system metabolising the effluent without prior treatment of the latter and combining, in a compatible manner, a strain of the first yeast belonging to the Candida kefyr species, metabolising the fermentable sugar while generating a by-product, in particular ethanol, and a strain of the second yeast belonging to the Candida valida species, metabolising the by-product without diauxie.

2. Method according to Claim 1, characterised in that the fermentable sugar belongs to the following list, namely lactose, glucose, galactose, sucrose, xylose, arabinose and fructose.

3. Method according to Claim 1, characterised in that the treated effluent contains, in addition, at least one compound belonging to the following list, namely ethanol, succinic acid, citric acid, lactic acid and glycerol.

4. Method according to Claim 1, characterised in that the culture is used in a fermenter, the biomass produced being separated by ultrafiltration, microfiltration or centrifugation.

5. Method according to Claim 1, characterised in that it comprises a stage of sequential discontinuous operation, consisting in setting up in the culture medium the first strain, then the second strain, followed by a culture stage, especially a continuous culture stage.

6. Method according to Claim 1, characterised in that the culture medium is used with at least one of the following specific conditions, namely:
   - the temperature is between 30°C and 38°C
   - the pH is between 2 and 4.5.

7. Method according to Claim 1, the organic effluent treated being a by-product of the dairy industry, such as a raw lactoserum or a raw dairy permeate, characterised in that the said effluent is brought into contact with the co-culture without prior treatment.

8. Yeast of the Candida kefyr species, identical to the strain deposited at the Collection Nationale de Micro-organismes at the Pasteur Institute, under reference I-747.

9. Yeast of the Candida valida species, identical to the strain deposited at the Collection Nationale de Micro-organismes at the Pasteur Institute under reference I-748.